# EUROPEAN PATENT APPLICATION

(11) **EP 2 236 067 A1**
(43) Date of publication of application: **06.10.2010**
(21) Application number: 09156856.8
(22) Date of filing: 31.03.2009
(51) Int. Cl.: A47K 7/03, A61K 8/02, A61K 8/11

(54) **Cleaning implement**

(71) Applicant: Unilever PLC, 100 Victoria Embankment London, Greater London EC4Y 0DY (GB); UNILEVER NV, 3013 AL Rotterdam (NL)
(72) Inventor: Crawford, Robert John, Wirral, Merseyside CH63 3JW (GB); Ferguson, Paul, Wirral, Merseyside CH63 3JW (GB); Hunter, Robert Allan, Wirral, Merseyside CH63 3JW (GB); Unali, Giovanni Francesco, Wirral, Merseyside CH63 3JW (GB)
(74) Representative: Mulder, Cornelis Willem Reinier

(57) **Abstract**

A cleansing implement comprising a generally planar compressed, water-swellable matrix and a solid cleansing composition coated on one face of the compressed matrix, the solid cleansing composition comprising an encapsulated perfume.

## Description

The present invention relates to a cleansing implement.

US 2005/0208111 discloses a dry disposable flexible article comprises water insoluble core layer made of cellulosic material between two water insoluble nonwoven layers comprised of synthetic fibres. It has perforations that assist in controlling water absorption into the core required for activating the cleaning solution and for the controlled and repeated release of the cleaning solution on its activation by the absorbed water.

EP 1 459 672 discloses a cleansing implement including a first outer layer; a second outer layer; and an inner layer disposed and retained between the outer layers, wherein at least one of the layers is water permeable, and the inner layer includes means for expanding the implement to at least about 1.5 times its original height.

A problem with certain pre-prepared cleansing items lies in the difficulty in providing them with suitable perfuming. In particular, in certain aspects of this invention, which comprise cleansing implements which comprise a generally planar compressed water-swellable matrix and a solid cleansing composition coated on one face of the compressed matrix, problems have been encountered in obtaining a lasting perfume effect for the product. In more detail, in embodiments in which a perfume is pre-mixed into the solid cleansing composition, the effect of this perfume is found to drop off quickly, resulting in a product the predominant smell of which is surfactant. This has been found to be particularly the case where the solid cleansing composition is in the form of a sheet.

A solution to this problem, which allows the product formulator to retain a relatively broad scope for perfume formulation, is to provide at least some of the perfume in the form of perfume encapsulates.

According to the present invention, there is provided a cleansing implement comprising a generally planar compressed, water-swellable matrix and a solid cleansing composition coated on one face of the compressed matrix, the solid cleansing composition comprising an encapsulated perfume.

Preferably, the implement is suitable for use on human skin.

Conveniently, the implement expands on contact with water, which may provide the consumer with a positive perception of the product. Where the matrix of the cleansing supplement is said to be compressed, it will have been compressed and retains its compressed state.

Advantageously, the implement comprises less than 10%, preferably less than 5% and especially preferably less than 2% wt. water.

Preferably, the cleansing composition comprises less 10%, preferably less than 5% and especially preferably less than 2% wt. water.

Conveniently, the matrix comprises a polysaccharide.

Advantageously, the matrix comprises a polysaccharide derived from beta-glucose.

Conveniently, the matrix is in the form of a sponge, preferably a reticulated sponge.

Preferably, the matrix comprises cellulose.

Conveniently, the matrix comprises chitosan.

Advantageously, the matrix comprises PVA.

Preferably, the implement is compressed from average depth A to average depth 0.01 to 0.5A, preferably 0.05 to 0.2A and most preferably from 0.08A to 0.12A.

Conveniently, a cleansing composition is impregnated into the matrix.

Advantageously, the cleansing composition comprises a surfactant selected from anionic, cationic, non-ionic and zwitterionic surfactants.

The solid cleansing composition according to the invention conveniently comprises a cast surfactant/polymer sheet of the type known in the art.

In one aspect, in the cleansing implement according to the invention the solid cleansing composition comprises an encapsulated perfume. Conveniently the product, for example the cleansing implement but preferably the solid cleansing composition, may additionally comprise a non-encapsulated perfume.

The perfume in the encapsulated perfume may be any suitable perfume. Conveniently the encapsulated perfume is selected so as to provide a pleasant fragrance to the product both when it is unwrapped prior to use, but also during use.

The perfume may be encapsulated in any known form of encapsulate, and by any encapsulation method. In some preferred embodiments the encapsulated perfume is in a core-shell encapsulate, as opposed to a matrix encapsulate.

However in other embodiments matrix encapsulates, encapsulated in for example spray dried starch, may be preferred. Conveniently, the encapsulate provides an average perfume droplet size of about 1 to 3,000 microns, conveniently 1 to 2,000 microns, conveniently 5 to 1,000 microns, conveniently 5 to 100 microns, conveniently about 5 to 50 microns. A suitable encapsulated perfume is encapsulated in a melamine formaldehyde shell, and has an average droplet size of around 5 to 20 microns, e.g. 10 microns.

Conveniently the encapsulate material is water-soluble.

Conveniently, the encapsulate material used in the encapsulated perfume is shear rupturing. Preferably, the encapsulate material in the encapsulated perfume is such that it is soft and deformable during manufacture, and stays soft and deformable whilst it is wet. However, on drying the encapsulate material becomes fragile and relatively brittle, which assists shear rupturing and subsequent perfume release. The encapsulate material is also preferably one which remains brittle even after re-wetting, which again assists perfume release.

Conveniently, the encapsulated perfume is mixed directly into a solution of the cleansing composition, which can then be applied to the implement. Where the solid cleansing composition is applied to the cleansing implement as a sheet, the encapsulated perfume can conveniently be added to a slurry of cleansing composition, and the cleansing composition cast as a film and dried, according to techniques known in the art. The sheet can then be applied to the cleansing implement.

In an alternative, a slurry (e.g. an aqueous slurry) of the encapsulated perfume can be applied to the outer surface of the solid cleansing composition, i.e. a surface of the solid cleansing composition not in contact with, or opposite the one which contacts the compressed matrix. In some embodiments the encapsulated perfume has been found to have a de-tackifying effect on the solid cleansing composition. As such, in some embodiments it has been found that an aqueous slurry of the encapsulated perfume may be unsuitable for attaching the solid cleansing composition (particularly in sheet form) to the implement. In a preferred embodiment the encapsulated perfume may be located either within the solid cleansing composition, or applied to a surface of the solid cleansing composition which is not in contact with the cleansing implement. Such a de-tackifying effect provided by the encapsulated perfume to a surface of the solid cleansing composition not in contact with the cleansing implement may also provide processing benefits.

Conveniently, the level of encapsulate perfume utilized is so as to result in a perfume level of around 0.2 wt % to 2 wt %, conveniently 0.5 wt % to 1.5 wt % of the combined encapsulated perfume/solid cleansing composition.

Preferably, the cleansing composition comprises a skin benefit agent.

Conveniently, the solid cleansing composition is in the form of a sheet.

Advantageously, the solid cleansing composition is coated on substantially all of the one face of the compressed matrix.

Conveniently, the implement is a pad.

Advantageously, the implement comprises a logo printed or embossed thereon.

According to another aspect of the invention, there is provided a package comprising a plurality of cleansing implements of the invention and outer wrapping, the outer wrapping being substantially water impermeable.

According to a further aspect of the invention, there is provided a method for producing a cleansing implement comprising providing a generally planar compressed water-swellable matrix and coating a solid cleansing composition on one face of the compressed matrix.

Preferably, water is applied to the compressed matrix, followed by coating the one face of the matrix with the solid cleansing composition.

Conveniently, water is applied to the solid cleansing composition before being coated on the one face of the compressed matrix.

Alternatively, the step of coating the solid cleansing composition onto the one face of the compressed matrix is performed without the addition of water.

Preferably, the step of coating the solid cleansing composition onto the one face of the matrix is performed after compressing the matrix. This may have benefits in terms of ease of application of the solid cleansing composition, for example by providing a smoother, more rigid and move continuous substrate with more move contact points for improved solid cleansing composition adhesion, as well as ease of printing.

In a further aspect, there may be provided a cleansing implement comprising a generally planar compressed, water-swellable matrix and a solid cleansing composition coated on one face of the compressed matrix.

Conveniently, the method further comprises the step of impregnating the matrix with a cleaning composition.

Alternatively, the method further comprises a drying step after the solid cleansing composition is coated onto the one face of the matrix.

The matrix of the cleansing implement is compressed and is water-swellable. Preferably, the matrix is compressed to from average depth A to average depth 0.01 to 0.5A, preferably 0.05 to 0.2A and most preferably from 0.08A to 0.12A.

Preferably, the compressed implement has a thickness of from 0.5 to 5mm and more preferably from 0.8 to 3mm. Preferably this is the average thickness along an axis in the general plane of the implement the axis passing through the implement's centre point on said plane.

Preferably, the fully expanded implement has a thickness of from 5 to 50mm and more preferably from 8 to 30mm. Preferably this is the average thickness along an axis in the general plane of the implement the axis passing through the implement's centre point on said plane.

Preferably, the implement is a pad and is a square, rectangle, circle, oval, ellipse or other such regular pattern in plan view.

The implement may also be irregularly shaped and have a varying thickness along said axis. For example, when expanded the implement may, in cross section, be circular, elliptical or oval in shape.

Preferably, the implement is suitable for use on human skin. By this is meant that it comprises materials which do not overly abrade the human skin.

Preferably, the implement comprises less than 10%, preferably less than 5% and especially preferably less than 2% wt. water. This prevents the implement from dimpling at the surface where water has contacted the product somewhere in the implement's body. This also allows the implement to retain an attractive, regular appearance, especially where the cleansing composition provides a glossy surface.

Preferably, the cleansing composition comprises less 10%, preferably less than 5% and especially preferably less than 2% wt. water.

Preferably, the matrix comprises a polysaccharide. More preferably, the polysaccharide is one derived from beta-glucose such as cellulose. Suitable sources of cellulose may also be used to make the matrix. Suitable sources include wood-pulp, cotton, hemp, jute and flax.

Conveniently, the matrix comprises chitosan or PVA.

Preferably, the cleansing composition is coated on and impregnated into the matrix.

Preferably, the cleansing composition comprises a surfactant selected from anionic, cationic, non-ionic and zwitterionic surfactants.

Preferably, the cleansing composition comprises a skin benefit agent. Suitable skin benefit agents include conditioners, moisturizers, exfoliants, sun-protection factors,

Preferably, the implement comprises a logo printed thereon. Printing is effected by any standard means such as ink-jet and laser printing.

A package comprising a plurality of cleansing implements as described in any preceding claim and outer wrapping, the outer wrapping being substantially water impermeable.

In a second aspect the present invention provides a method for making an implement according to the first aspect, the method comprising presenting a suitably dimensioned implement as described in the first aspect of the invention, compressing it and then applying to it a cleansing composition comprising less than 10% wt. water.

The implement may be compressed following any standard process for example rolling or pressing.

The cleansing composition may also be applied prior to compressing.

The cleansing composition in solid form is coated onto one surface of the generally planar compressed matrix. The solid cleansing composition can be in the form of a sheet or may have other shapes.

Preferably, the solid cleansing composition is coated on the matrix such that it substantially covers one face of the compressed matrix. In other embodiments, the solid cleansing composition is coated onto the one face of the matrix such that it covers less than the total surface area of the one face.

In other words, one of the two faces or sides of the compressed matrix is coated with the solid cleansing composition, whilst the other face or side of the compressed matrix is substantially free of the solid cleansing composition.

In addition to being coated on one face of the matrix, a cleansing composition may additionally added to the matrix in a liquid form, such as a solution or suspension.

Preferably the cleaning implement comprises a generally planar water-swellable compressed matrix having a solid cleansing composition coated on one face in addition to a cleansing composition impregnated in the matrix.

Preferably, the cleansing composition comprises all the cleansing components and optionally any benefit agent components all dissolved or suspended in a nonaqueous solvent such as ethanol.

The solid cleansing composition may be coated onto the one face of the matrix as follows. In a preferred embodiment, a previously prepared solid cleansing composition is contacted with sufficient water to enable it to be contacted with and coated on the one face of the matrix. Alternatively, the matrix may itself be contacted with sufficient water to enable it to be coated with the solid cleansing composition. Furthermore, both the matrix and the solid cleansing composition may be contacted with water before being brought together.

In a preferred aspect, the cleansing implement is a synthetic reticulated sponge. The sponge may be made by known techniques, and then conveniently in a continuous process subsequently compressed and heated. The compressed and heated sponge retains its compressed state; whilst still at elevated temperature the solid cleansing composition may then be applied, for example in the form of a sheet, which is conveniently moistened on the face which contacts the sponge, prior to its application to the sponge.

In another preferred embodiment, the solid cleansing composition is prepared such that it retains sufficient tackiness, for example by having a sufficiently high level of water, to enable it to be contacted with and coated onto one face of the matrix. In this way, the solid cleansing composition may be prepared and attached to the matrix in a continuous or semi-continuous manner.

Preferably, the matrix does not contain any binder or other material to help it retain its compressed form. In a preferred embodiment, the matrix comprises cellulose and does not contain any such binder.

The matrix may also have applied during manufacture, for example by spraying on or rolling on, other layers of composition which do not cause swelling of the compressed matrix. Such additional layers of composition may be in the form of non-swelling slurries or pastes. Such an additional layer could be for example a water barrier layer located on the matrix between the compressed matrix and the solid cleansing composition. The provision of a water barrier layer would allow for the solid cleansing composition to be slightly damp during manufacture when applied, and also to prevent migration of components.

The cleansing composition can also be applied to the compressed sponge by taking a dry-sheet of cleanser such as those used by campers (Leafz®), misting with water and then applying to the compressed sponge. There is sufficient water in the misting process to slightly expand the sponge without changing its overall dimension and in the process stick to the sheet of cleanser. The cleansing implement may then be used by the consumer.

In a further embodiment, especially where the solid cleansing composition is in the form of a sheet, the solid cleansing composition may be applied with an adhesive which could be applied to either the matrix or the solid cleansing composition.
The adhesive could be a water-soluble adhesive such as PVA, a starch-based adhesive or a dextrin-based adhesive, a polysaccharide adhesive such as e.g. a cellulose ether adhesive, or a clay-based adhesive. Alternatively the adhesive could be a pressure-sensitive adhesive. In other embodiments, a non-water soluble adhesive may be preferred.

The cleansing composition according to the invention may also be supplemented with any of the following actives: Anti-acne, anti-wrinkle, tanning agents, anti-microbial actives, anti-fungal actives, sunscreens, cosmetics, vitamins, anti-cellulite actives, skin-lightening agents, antiperspirants, anti-virals, exothermic zeolites, exfoliants, fragrances, conditioning agents and mixtures thereof.

The present invention provides a number of important advantages. Firstly, providing the compressed matrix with a solid cleansing composition on one face leaves the other face of the matrix in a condition to be easily held and retained by a user. In other words, if the implement is being used in a shower, the user can hold one face of the implement in their hand which is not coated with a potentially slippery cleansing composition.

Also, the user is able to directly feel the expansion of the matrix when the cleansing implement is contacted with water.

Another advantage is that the solid cleansing composition on face of the matrix is available for immediate use by the user. In other words, the implement may be immediately used for cleansing purposes by the user upon contact with water, which may not be the case if the cleansing composition was not provided on the outer surface of the implement.

The structure also allows for inexpensive and simple manufacture. In particular, the sponge and the solid cleansing composition may be manufactured separately, potentially at different times and at different sites. The compressed matrix and the solid composition may then be brought together and readily adhered. The coating process can be easy to perform by misting at least one of the matrix and the solid coating composition with an aqueous liquid and thereafter bringing them together.

An alternative preferred method of preparing the implement of the invention is to prepare the solid cleansing composition, leaving it with sufficient tackiness to enable it to be directly coated onto one face of the matrix. For example, the solid coating composition may be prepared containing a sufficiently high level of water to give it enough tackiness for it to be contacted and coated onto one face of the matrix.

The cleaning implements may be used in personal care applications, for example as a cleansing implement to be used by a user in the shower. They may also or alternatively be used in other forms of cleansing, such as hard surface applications.

A user of the implement in, for example, a shower may use the cleaning implement to clean the surfaces of the shower area after using it for personal cleansing.

### EXAMPLE 1

A cellulose sponge was compressed to 10% of its original thickness with a press. A standard shower-gel composition was dried in the oven and dissolved in ethanol. The dried shower-gel composition in ethanol was then applied to the surface of the compressed sponge and then oven-dried.

The sponge can be activated by dunking in water.

### EXAMPLE 2

A cellulose sponge was compressed to 10% of its original thickness with a press. A sheet of cleansing composition (solid soap) was misted with a small amount of water. The sheet was then pressed into contact with one face of the sponge.

The sponge can be activated by dunking in water.

### EXAMPLE 3

A cellulose reticulated sponge was compressed to 10% of its original thickness with pressure and heat. A sheet of solid cleansing composition, comprising a mixture of synthetic surfactants and polymer and additionally comprising 1% free perfume, was misted with a small amount of water; the sheet was then pressed into contact with one face of the sponge and dried.

The sponge could be activated by immersing it in water. After one week, in a fume cupboard at 20-25°C, all perfume scent had disappeared from the implement.

A similar implement was prepared, only instead of 1% free/unencapsulated perfume, an encapsulated perfume was utilized at a level which resulted in a level of 1% by weight of perfume in the cleansing composition. After one week in a fume cupboard at 20-25°C, the implement had a scent of perfume; furthermore this perfume was noticeable after the sponge was activated with water.

## Claims

1. A cleansing implement comprising a generally planar compressed, water-swellable matrix and a solid cleansing composition coated on one face of the compressed matrix; the solid cleansing composition comprising an encapsulated perfume.

2. An implement according to claim 1 suitable for use on human skin.

3. An implement according to claim 1 or 2 which expands on contact with water.

4. An implement according to any preceding claim comprising less than 10%, preferably less than 5% and especially preferably less than 2% wt. water.

5. An implement according to any preceding claim wherein the cleansing composition comprises less 10%, preferably less than 5% and especially preferably less than 2% wt. water.

6. An implement according to any preceding claim wherein the matrix comprises a reticulated synthetic sponge.

7. An implement according to any preceding claim which is compressed from average depth A to average depth 0.01 to 0.5A, preferably 0.05 to 0.2A and most preferably from 0.08A to 0.12A.

8. An implement according to any preceding claim wherein the solid cleansing composition is in the form of a sheet.

9. An implement according to any preceding claim wherein the solid cleansing composition is coated on substantially all of the one face of the compressed matrix.

10. An implement according to any one of the preceding claims, wherein the solid cleansing composition comprises a non-encapsulated perfume.

11. An implement according to any one of the preceding claims, wherein the encapsulated perfume is a core-shell encapsulate.

12. An implement according to any one of the preceding claims, wherein the encapsulated perfume provides a perfume droplet size of 5 to 50 microns.

13. An implement according to any one of the preceding claims, wherein the encapsulated perfume is encapsulated in an encapsulate material which remains brittle when it is re-wetted.

14. An implement according to any one of the preceding claims, wherein the encapsulated perfume is encapsulated in a melamine formaldehyde shell.

15. An implement according to any one of the preceding claims, wherein the encapsulated perfume is either within the solid soap composition or applied to a surface of the solid cleansing composition which is not in contact with the cleansing implement.
